# EUROPEAN PATENT APPLICATION

(11) **EP 3 449 853 A1**
(43) Date of publication of application: **06.03.2019**
(21) Application number: 18190664.5
(22) Date of filing: 24.08.2018
(51) Int. Cl.: A61B 17/34

(54) **ACCESS APPARATUS INCLUDING SEAL COMPONENT WITH PROTECTIVE GUARDS**

(30) Priority: 28.08.2017 US 201762550752 P; 24.07.2018 US 201816043551
(71) Applicant: Covidien LP, Mansfield, MA 02048 (US)
(72) Inventor: HOLSTEN, Henry E., Hamden, CT 06518 (US)
(74) Representative: Maschio, Antonio

(57) **Abstract**

A seal component for use with a cannula assembly of the type including a housing and a cannula sleeve configured for accessing an underlying body cavity and defining a longitudinal passage for introduction of a surgical object is provided. The seal component includes an object seal member mounted with respect to the housing and defining a seal passage configured to sealingly engage a surgical object and at least a first guard member proximal of the seal member. The first guard member includes a first sheet of material defining a first guard aperture in general longitudinal alignment with the seal passage of the seal member. The first sheet of material is configured to be engaged by the surgical object to protect the object seal member during passage of the surgical object. In embodiments, the first sheet of material comprises Tyvek®.

## Description

### CROSS-REFERENCE TO RELATED APPLICATIONS

This application claims the benefit of and priority to U.S. Provisional Patent Application Serial No. 62/550,752 filed August 28, 2017, the entire disclosure of which is incorporated by reference herein.

### BACKGROUND

### 1. Technical Field

The present disclosure relates to an access apparatus for accessing a body cavity. More particularly, the present disclosure relates to a seal component for use with an access apparatus and having at least one protective guard to minimize the potential of inadvertent puncture or damage to an object or instrument seal of the seal component upon insertion of a surgical object.

### 2. Background of Related Art

Minimally invasive surgical procedures including both endoscopic and laparoscopic procedures permit surgery to be performed on organs, tissues and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula accessing the abdominal cavity to perform one or more surgical tasks. The cannula may incorporate a seal to establish a substantially fluid tight seal about the instrument to preserve the integrity of the pneumoperitoneum.

Minimally invasive procedures have several advantages over traditional open surgery, including less patient trauma, reduced recovery time, reduced potential for infection, etc. However, despite its success and overall acceptance as a preferred surgical technique, minimally invasive surgery, such as laparoscopy, has disadvantages. For example, many conventional seals within cannulas are subject to damage and/or degradation during introduction and manipulation of the surgical instrument within the cannula. This may result in an ineffective seal created about the inserted surgical instrument with a potential loss of insufflation gases within the peritoneal cavity.

### SUMMARY

Accordingly, the present disclosure is directed to a seal component for use with an access apparatus, which incorporates an object seal with protective guard element(s) to preserve the integrity of the seal and protect the seal from damage during introduction of a surgical object. In one exemplary embodiment, a seal component for use with a cannula assembly is provided. The cannula assembly is of the type including a housing and a cannula sleeve configured for accessing an underlying body cavity and defining a longitudinal axis and having a longitudinal passage for introduction of a surgical object. The seal component includes an object seal member mounted with respect to the housing and defining a seal passage configured to sealingly engage a surgical object, and at least a first guard member proximal of the seal member. The first guard member includes a first sheet of material defining a first guard aperture in general longitudinal alignment with the seal passage of the seal member. The first sheet of material is configured to be engaged by the surgical object to protect the seal member during passage of the surgical object. In embodiments, the first sheet of material comprises Tyvek®. In the alternative, the first sheet of material comprises one of plastic or paper.

In embodiments, the seal passage of the seal member is a seal orifice. In some embodiments, the first guard aperture of the first guard member defines an internal dimension greater than a corresponding internal dimension of the seal orifice of the seal member. In certain embodiments, the first guard member includes a plurality of first radial slots coterminous with the first guard aperture and extending radially outwardly with respect to the longitudinal axis.

In some embodiments, a second guard member is proximal of the first guard member. The second guard member includes a second sheet of material defining a second guard aperture in general alignment with the seal orifice of the seal member. The second sheet of material may comprise Tyvek®. In the alternative, the second sheet of material comprises one of plastic or paper. In certain embodiments, the second guard member includes a plurality of second radial slots coterminous with the second guard aperture and extending radially outwardly with respect to the longitudinal axis. In some embodiments, the second radial slots of the second guard member are radially offset with respect to the first radial slots of the first guard member.

The Tyvek® material of the first and second sheets of the respective first and second guard members permit passage of the instrument without tearing of its material thereby preserving the integrity of the underlying seal member. The radial slots permit the apertures of the first and second guard members to readily expand without resisting traversing movement of the surgical object through the guard members. The flexibility and sheet-like qualities of the first and second guard members will not inhibit offset movement of the surgical object during performance of the surgical task.

Other advantages of the seal component will be appreciated from the following description.

### BRIEF DESCRIPTION OF THE DRAWINGS

Various aspects and features of the present disclosure are described hereinbelow with references to the drawings, wherein:
FIG. 1 is a perspective view illustrating a cannula assembly and a seal assembly incorporating the seal component of the present disclosure with the seal assembly shown separated from the cannula assembly;
FIG. 2 is an exploded perspective view of the seal assembly illustrating the upper housing component, the seal component and the lower housing component;
FIG. 3 is an exploded perspective view of the seal component illustrating the seal retainers, the object seal member, the centering element and the first and second guard members for protecting the seal member; and
FIG. 4 is an enlarged cross-sectional view of the seal component of the seal assembly.

### DETAILED DESCRIPTION

Particular embodiments of the present disclosure are described hereinbelow with reference to the accompanying drawings. However, it is to be understood that the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to employ the present disclosure in virtually any appropriately detailed structure.

The present disclosure relates to a seal component which may be incorporated with an access apparatus, such as a cannula assembly, and includes an object seal member and at least one protective guard to prevent inadvertent damage, tearing, puncture etc. to the seal member during introduction and manipulation of a surgical object such as a surgical instrument during a laparoscopic procedure. The at least one protective guard may be fabricated from a thin flexible sheet of material such as Tyvek®. The thin protective guard provides adequate protection to the seal member during use of the instrument and exhibits sufficient flexibility to not interfere with the functioning of the seal member nor restrict manipulation of the surgical instrument. In embodiments, two protective guards are included and arranged in superposed relation with respect to the seal member. The protective guards may incorporate radial slots which are offset with respect to each other to facilitate passage of the instrument through the protective guards while preserving the underlying pneumoperitoneum.

The following discussion will focus on the use of the seal component, including the object seal member and protective guards, as components of a seal assembly for use with an access apparatus, e.g., a cannula assembly. However, it is to be appreciated that the seal component may be directly incorporated within the cannula assembly.

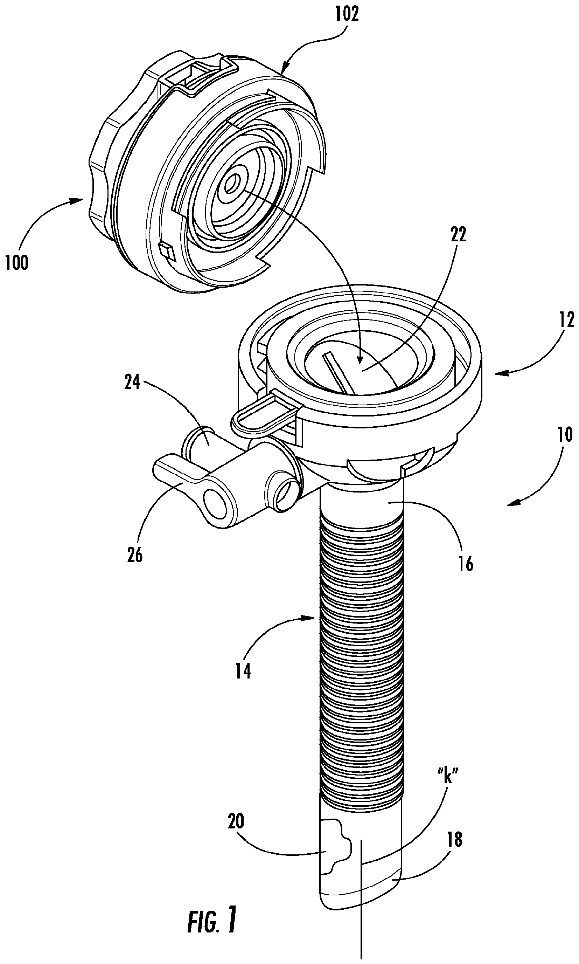

Referring initially to FIG. 1, there is illustrated an access assembly 10 incorporating a seal assembly 100 having the seal component of the present disclosure. In embodiments, the access assembly is a cannula assembly 10 intended to permit access to an insufflated peritoneal cavity during a laparoscopic procedure to permit the introduction of a surgical object for performing various surgical tasks on internal organs within the cavity. The surgical object may be a surgical instrument such as laparoscopic or endoscopic clip appliers, graspers, dissectors, retractors, staplers, laser probes, photographic devices, tubes, endoscopes and laparoscopes, electro-surgical devices, and the like. The cannula assembly 10 includes a cannula housing 12 and a cannula sleeve 14 extending from the cannula housing 12. The cannula sleeve 14 defines proximal and distal ends 16, 18 and a longitudinal axis "k" extending along the length of the cannula sleeve 14. The cannula housing 12 and the cannula sleeve 14 define a longitudinal opening 20 (cut-away in FIG. 1) for reception and passage of the surgical object. The cannula housing 12 may include a zero closure valve 22, e.g., a duckbill valve, which is configured to close in the absence of a surgical object to prevent egress of insufflation gases. The zero closure valve 22 does not typically establish a seal about an inserted surgical object. The cannula housing 12 also includes an insufflation port 24 and associated insufflation valve 26 (e.g., a stop cock valve) for selective introduction of insufflation fluids into the cannula sleeve 14 and the peritoneal cavity. Further details of a cannula assembly 10 for use with the seal assembly 100 may be ascertained by reference to commonly assigned U.S. Publication No. 2015/0216560 to Holsten, the entire contents of which is hereby incorporated by reference herein.

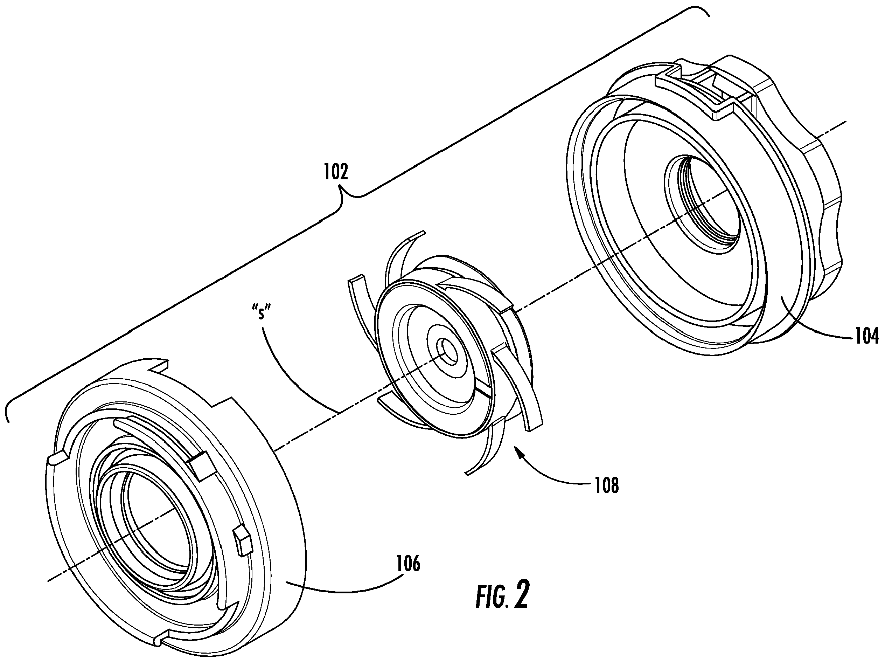

With reference to FIGS. 1-2, the seal assembly 100 may be selectively mountable to the cannula assembly 10 to provide sealing capabilities, e.g., to establish a sealing relation about an inserted surgical object. Any mechanism for releasably mounting the seal assembly 100 to the cannula housing 12 is envisioned including, e.g., a friction fit, bayonet coupling, snap fit, and the like. The seal assembly 100 includes a seal housing, identified generally as reference numeral 102, defining a seal axis "s" (FIG. 2) which is in general longitudinal alignment with the longitudinal axis "k" of the cannula sleeve 14 in the assembled condition of the components. The seal housing 102 includes a proximal housing component 104 and a distal housing component 106. In the alternative, the seal housing 102 may be a single component monolithically or integrally formed to incorporate the proximal and distal housing components 104, 106.

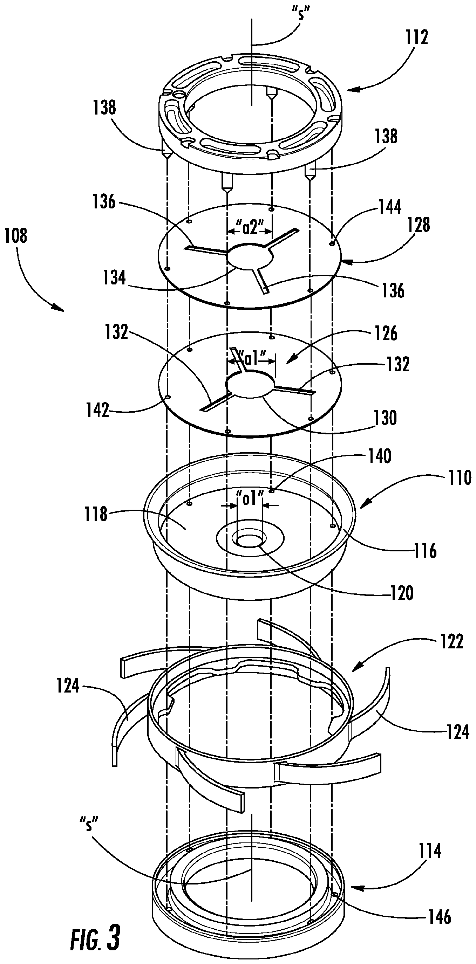

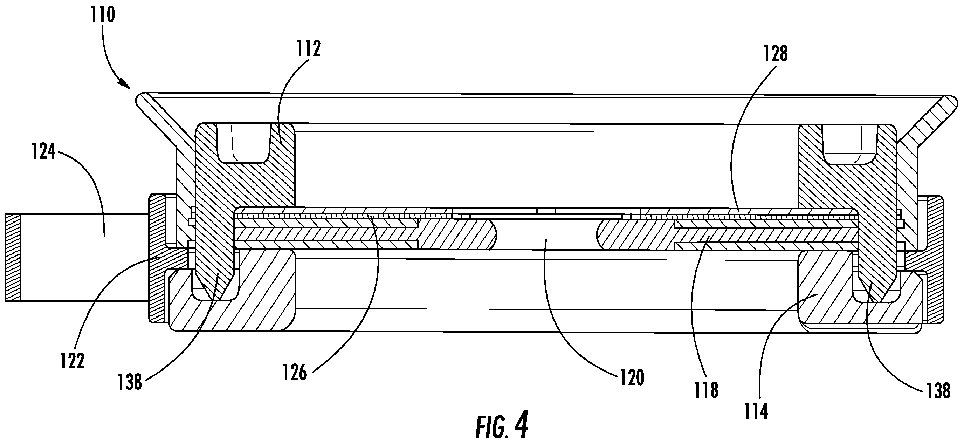

With reference to FIGS. 2-4, the seal assembly 100 includes a seal component 108 having an object seal member 110, an upper seal retainer 112, and a lower seal retainer 114. The seal member 110 includes an outer seal flange or collar 116 and an inner seal portion 118 depending radially inwardly from the seal collar 116. At least the inner seal portion 118 may be fabricated, at least in part, from an elastomeric material, and may have one or more fabric layers positioned on, or embedded within, the elastomeric material. The inner seal portion 118 defines a seal passage 120 for reception and passage of a surgical object in sealed relation therewith. In embodiments, the seal passage 120 is an open seal orifice defining an orifice dimension "ol". A centering collar 122 is disposed about the periphery of the seal member 110 and has a plurality of resilient spokes 124 depending outwardly from the centering collar 122. The spokes 124 bias the seal member 110 through engagement with the interior of the seal housing 102 to a position where the seal passage 120 is in general alignment with the seal axis "s", and may minimize offset movement of the seal member 110 during manipulation of the surgical object.

The seal component 108 further at least one protective guard member, e.g., first and second protective guard members, 126, 128 arranged in superposed relation with respect to the seal member 110. In particular, the first and second guard members 126, 128 are dimensioned to reside within the seal collar 116 of the seal member 110 and are confined therein within the outer boundary defined by the seal collar 116. The first guard member 126 is proximal of and in contacting relation with the inner seal portion 118 of the seal member 110. The first guard member 126 includes a first guard aperture 130 and a plurality of first radial slots 132 extending outwardly from the first guard aperture 130 and coterminous therewith. The first radial slots 132 may be equidistantly radially spaced with respect to the seal axis "s". Similarly, the second guard member 128 includes a second guard aperture 134 and a plurality of second radial slots 136 extending outwardly from the second guard aperture 134 and coterminous therewith. The second radial slots 136 may be equidistantly radially spaced with respect to the seal axis "s". The first and second radial slots 132, 136 permit expansion of the respective first and second guard apertures 130, 134 thereby facilitating passage of the surgical object through the first and second guard members 126, 128. In embodiments, the first radial slots 132 of the first guard member 126 are radially offset with respect to the second radial slots 136 of the second guard member 128. This displacement of the first and second radial slots 132, 136 minimizes the potential of loss of insufflation gases through the guard members 126, 128, e.g., and thus through the seal component 108 during insertion and/or offset manipulation of the surgical object. The first and second guard apertures 130, 134 define respective aperture dimensions "a1", "a2" which are greater than the orifice dimension "01" of the seal member 110 to not interfere or grab the surgical object as it passes through the seal passage or orifice 120. Apertures and/or slots of other shapes can be used. In certain embodiments, the guard members have apertures and/or slots of a particular shape, and are assembled off-set from one another by 45 to 100 degrees.

The first and second guard members 126, 128 are configured to be engaged by the surgical object to protect the seal member 110 during passage of the surgical object. In embodiments, the first and second guard members 126, 128 have sufficient flexibility to not interfere with passage of the surgical object and also possess sufficient strength to adequately protect the underlying seal member 110 from damage during e.g., insertion of a pointed instrument. The first and second guard members 126, 128 are each fabricated from respective first and second sheets of material. The first and second guard members 126, 128 may include first and second sheets of material fabricated from Tyvek® which is manufactured by DuPont of Wilmington, DE. Tyvek® is a nonwoven product consisting of spunbond olefin fibers, and is characterized by being very strong, tear-resistant, chemical resist and dimensionally stable. These characteristics enable the first and second guard members 126, 128 to be relatively thin thereby reducing the overall profile of the seal component 108 while satisfying the aforedescribed objectives of strength, flexibility durability, etc... Alternatively, the first and second sheets of material of the first and second guard members 126, 128 may be fabricated from one of plastic or paper. The material can include high density polyethylene fibers, mesh of some other polymer, nonwoven materials, etc.

The seal member 110 and the first and second guard members 126, 128 are retained within the upper and lower seal retainer 112, 114 by a plurality of pins 138 extending from the upper seal retainer 112 and extending through corresponding respective peripheral apertures 140, 142, 144 of the seal member 110 and the first and second guard members 126, 128 for reception within apertures 146 of the lower seal retainer 114. Other methodologies for securing the seal member 110 and the first and second guard members 126, 128 are also envisioned.

In use, the cannula assembly 10 is introduced within the peritoneal cavity with the use of an obturator positioned through the cannula assembly 10. The obturator is removed and a surgical object is passed through the seal assembly 100. During passage of the surgical object, the first and second guard members 126, 128 engage the end of the surgical object to protect the underlying seal member 110 from damage and/or puncture. As previously mentioned, if fabricated from Tyvek® or a material having similar properties, the first and second guard members 126, 128 permit passage of the object without tearing of its material thereby preserving the integrity of the underlying seal member 110. The radial slots 132, 136 permit the apertures 130, 134 of the first and second guard members 126, 128 to readily expand without resisting traversing movement of the surgical object through the guard members 126, 128. In addition, due to the enlarged internal dimensions "a1", "a2" of the apertures 130, 134 of the first and second guard members 126, 128 relative to the orifice dimension "01" of the seal member 110, the object may pass through the first and second guard members 126, 128 and the seal passage or orifice 120 of the seal member 110 without the sheets snagging or grabbing the surgical object. The flexibility and sheet-like qualities of the first and second guard members 126, 128 will not inhibit offset movement of the surgical object during performance of the surgical task.

Although the illustrative embodiments of the present disclosure have been described herein with reference to the accompanying drawings, the above description, disclosure, and figures should not be construed as limiting, but merely as exemplifications of particular embodiments. For example, the seal unit may be utilized in other capacities such as, e.g., in hand access systems where the surgeon's hand is introduced within the peritoneal cavity to assist in performing the laparoscopic procedure. The seal assembly may be contemplated for use in surgical procedures in other areas of the body, e.g., in other endoscopic procedures including arthroscopic, gynecological, spinal procedures, and the like. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A seal component for use with a cannula assembly including a housing and a cannula sleeve configured for accessing an underlying body cavity and defining a longitudinal axis and having a longitudinal passage for introduction of a surgical object, the seal component comprising:
   an object seal member mounted with respect to the housing, the seal member defining a seal passage configured to sealingly engage a surgical object; and
   at least a first guard member proximal of the seal member, the first guard member including a first sheet of material defining a first guard aperture in general longitudinal alignment with the seal passage of the seal member, the first sheet of material configured to be engaged by the surgical object to protect the seal member during passage of the surgical object.
2. The seal component according to paragraph 1 wherein the first sheet of material of the first guard member comprises Tyvek®.
3. The seal component according to paragraph 1 wherein the first sheet of material of the first guard member comprises one of plastic or paper.
4. The seal component according to clam 1 wherein the seal passage of the seal is a seal orifice.
5. The seal component according to paragraph 4 wherein the first guard aperture of the first guard member defines an internal dimension greater than a corresponding internal dimension of the seal orifice of the seal member.
6. The seal component according to paragraph 5 wherein the first guard member includes a plurality of first radial slots coterminous with the first guard aperture and extending radially outwardly with respect to the longitudinal axis.
7. The seal component according to paragraph 6 including a second guard member proximal of the first guard member, the second guard member including a second sheet of material defining a second guard aperture in general alignment with the seal orifice of the seal member.
8. The seal component according to paragraph 7 wherein the second sheet of material of the second guard member comprises Tyvek®.
9. The seal component according to paragraph 7 wherein the second sheet of material of the second guard member comprises one of plastic or paper.
10. The seal component according to paragraph 7 wherein the second guard member includes a plurality of second radial slots coterminous with the second guard aperture and extending radially outwardly with respect to the longitudinal axis.
11. The seal component according to paragraph 10 wherein the second radial slots of the second guard member are radially offset with respect to the first radial slots of the first guard member.

## Claims

1. A seal component for use with a cannula assembly including a housing and a cannula sleeve configured for accessing an underlying body cavity and defining a longitudinal axis and having a longitudinal passage for introduction of a surgical object, the seal component comprising:
an object seal member mounted with respect to the housing, the seal member defining a seal passage configured to sealingly engage a surgical object; and
at least a first guard member proximal of the seal member, the first guard member including a first sheet of material defining a first guard aperture in general longitudinal alignment with the seal passage of the seal member, the first sheet of material configured to be engaged by the surgical object to protect the seal member during passage of the surgical object.

2. The seal component according to claim 1 wherein the first sheet of material of the first guard member comprises Tyvek®.

3. The seal component according to claim 1 or claim 2 wherein the first sheet of material of the first guard member comprises one of plastic or paper.

4. The seal component according to any preceding claim wherein the seal passage of the seal is a seal orifice.

5. The seal component according to claim 4 wherein the first guard aperture of the first guard member defines an internal dimension greater than a corresponding internal dimension of the seal orifice of the seal member.

6. The seal component according to claim 5 wherein the first guard member includes a plurality of first radial slots coterminous with the first guard aperture and extending radially outwardly with respect to the longitudinal axis.

7. The seal component according to claim 6 including a second guard member proximal of the first guard member, the second guard member including a second sheet of material defining a second guard aperture in general alignment with the seal orifice of the seal member.

8. The seal component according to claim 7 wherein the second sheet of material of the second guard member comprises Tyvek®.

9. The seal component according to claim 7 wherein the second sheet of material of the second guard member comprises one of plastic or paper.

10. The seal component according to any of claims 7 to 9 wherein the second guard member includes a plurality of second radial slots coterminous with the second guard aperture and extending radially outwardly with respect to the longitudinal axis.

11. The seal component according to claim 10 wherein the second radial slots of the second guard member are radially offset with respect to the first radial slots of the first guard member.
